Europäisches Patentamt

European Patent Office

Office européen des brevets

(11) Publication number: 0 248 531
A2

# (12) EUROPEAN PATENT APPLICATION

(21) Application number: 87303955.6

(22) Date of filing: 01.05.87

(51) Int. Cl.⁴: **A61K 9/50** , **A61K 9/16** ,
**A61K 31/70**

(30) Priority: 02.05.86 US 859019

(43) Date of publication of application:
09.12.87 Bulletin 87/50

(84) Designated Contracting States:
AT BE CH DE ES FR GB GR IT LI LU NL SE

(71) Applicant: **Southern Research Institute
2000 Ninth Avenue South
Birmingham Alabama 35205(US)**

(72) Inventor: **Tice, Thomas R.
1305 Overland Drive
Birmingham Alabama 35216(US)**
Inventor: **Pledger, Kenneth L.
955D Beacon Parkway East
Birmingham Alabama 35209(US)**
Inventor: **Gilley, Richard M.
4020 Royal Oak Circle
Birmingham Alabama 35243(US)**

(74) Representative: **Perry, Robert Edward et al
GILL JENNINGS & EVERY 53-64 Chancery
Lane
London WC2A 1HN(GB)**

(54) **Encapsulated nucleic acids.**

(57) RNA and/or DNA, e.g. poly(I:C) or anti-sense
RNA, is encapsulated as microcapsules in a biodeg-
radable, biocompatible polymeric material, for the
controlled release of the nucleic acid on administra-
tion and the induction of interferon production.

EP 0 248 531 A2

Xerox Copy Centre

# ENCAPSULATED NUCLEIC ACIDS

This invention relates to encapsulated nucleic acids.

Interferon is a potential inhibitor of viral replication. It is a host-specific protein naturally secreted by organisms exposed to viral agents, see Isaacs, A. Advan. Virus Res. 10,1(1963) and Ho, M. in "Interferons" (N.B.Finter, ed.), Chapter 2, pp 21-54 North-Holland Publ., Amsterdam (1967). More specifically, after exposure to viral agents, organisms synthesize interferon from messenger RNA transcribed from a host gene; see Wagner, R.R., Huang, A.S. Virology 28, 1 (1963); Heller, E. Virology 21, 652 (1963) and Wagner, R. R. Trans. Ass. Am. Physicians 76, 92 (1963). Interferon is species specific, but biologically active against a variety of viruses; see Buckler, C.E., Baron, S. J. Bacteriol 91, 231 (1966) and Merigan, T.C. Science 145, 811 (1964).

Because of the potential therapeutic value of protecting an animal from a variety of viral infections by the animal's own interferon system, there has been research concentrated on finding non-viral agents capable of inducing the production of interferon; see Ho, M., Postic, B., Ke, Y.H. in "Ciba Foundation Symposium on Interferon" (Wolstenholme and O'Connor, ed.), p. 19, Little, Brown, Boston, Mass., USA (1967) and Ho, M., Fantes, K.H., Burke, D.C., Frater, N.B. in "Interferons" supra p.181. In particular, a complex of polyriboinosinic-polyribocytidylic acid, also known as poly(I:C), has been shown to be a potent inducer of interferon when administered in low doses; see Field, A.K., Tytell, A. A., Lampson, G.P. Hilleman, M.R. Proc. Nat. Acad. Sci. 58, 1004-1010 (1967); Parks J.H., Baron, S. Science 162, 811-813 (1968); Wothington, M., Levy, H., Rice, J. Proc. Soc. Exp. Biol. Med. 143, 638-643 (1973); Zeleznick, L.D., Bhuyan, B.K. Proc. Soc. Exp. Biol. Med. 130, 126-128 (1969); Levy, H.B., Law, L.W., Rabson, A.S. Proc. Nat. Acad. Sci. 62, 357-361 (1969) and Gelboin, H., Levy, H.B. Science 167, 205-207 (1970).

Major disadvantages associated with the use of poly(I:C) to induce interferon production include its toxicity at high doses and its short half-life in humans. The short half-life is due to the fact that human serum contains a high concentration of a nucleolytic enzyme complex that rapidly hydrolyzes, and thereby inactivates, poly(I:C); see Nordlund, J.J., Wolff, S.M., Levy, H.B. Proc. Soc. Exp. Biol. Med. 133, 439-444 (1970). To overcome the problems associated with poly(I:C) administration, most researchers have developed multiple dosing schedules that require repetitive injections of small quantities of poly(I:C) for periods of four to seven days; see Kends, M. J. Biol. Response Mod. 4, 503-511 (1985); Stephen, E.L., Bammons, M.L., Pannier, W.L., Barron, S., Spertzel, F.O., Levy, H.G. J. Infect. Dis. 136, 122-126 (1977); Levy, H.B., Baer, G., Baron, S., et al. J. Infect. Dis. 132, 434-439 (1975) and Sammons, M.L., Stephen, E.L., Levy, H.B., Baron, S., Himas, D.E. Antimicrob. Agents Chemother. 11, 80-83 (1977).

RNA also has a short half-life in the body; see Klausner, A. Bio/Technology 3, 763-764 (1985) and Izant, J.G., Weintraub, H. Science 229, 345-352 (1985).

According to the present invention, controlled-release microcapsules comprise RNA or DNA, or a mixture thereof, encapsulated in a biocompatible, biodegradable polymer. After a single administration, particularly by injection, e.g. parenterally, the microcapsules release the RNA or DNA at a controlled rate, resulting in induction of high titers of interferon. The microcapsules protect the nucleic acid from enzymatic digestion or degradation before it is released.

The present invention provides a pharmaceutical composition designed for the controlled release or sustained release of an effective amount of RNA and/or DNA, over an extended period of time; the RNA or DNA is encapsulated in a biocompatible, biodegradable polymeric material which protects the nucleic acid from enzymatic degradation. The microcapsules can have an internal structure whereby a single reservoir of RNA or DNA is coated with a polymeric membrane; alternatively, the microcapsules can have an internal structure whereby the RNA or DNA is dispersed within a polymeric matrix.

The term biocompatible may be defined as non-toxic to the human body, non-carcinogenic and non-inflammatory in body tissues. The matrix material should be biodegradable in the sense that the polymeric material should degrade by bodily processes to products readily disposable by the body, and should not accumulate in the body. The biodegraded products also should be biocompatible with the body in the sense that the polymeric matrix is compatible with the body.

A particular application for the controlled release of RNA pertains to the delivery of anti-sense RNA to the body, to turn off the expression of specific genes. It has been shown that gene expression can be turned off at the translation level. More specifically, translation can be blocked by introducing into cells RNA that is complementary to a particular message RNA produced in that cell. The complementary RNA (anti-sense RNA) will hybridize with the message RNA and prevent the synthesis of the protein coded on the message RNA. Because of the short half-life of RNA in the body, a practical way of delivering anti-sense RNA for therapeutic treatment is by releasing it from biocompatible, biodegradable microcapsules.

The excipient for the nucleic acid microcapsules can be a synthetic polymer. Examples of the polymeric excipients suitable for use in the present invention include poly(D,L-lactide), poly(glycolic acid), copolymers of mixed D,L-lactide and glycolide, copolyoxalates, polycaprolactone, poly-(lactide-co-caprolactone), poly(esteramides) and poly(beta-hydroxybutyric acid).

The nucleic acid can be a synthetic or naturally-produced species. The RNA and/or DNA may be either single-stranded or double-stranded.

Microcapsules of the present invention can be prepared by any method which is capable of producing microparticles in a size and range acceptable for use in an injectable composition. The microcapsules can be loaded with from 0.01 to 75 wt.% of the RNA or DNA, based on the polymeric matrix. The microcapsules may range in size from 0.1 to 500 $\mu$m. In the treatment of animals, the microcapsules of the present invention are dispersed in a pharmaceutically acceptable carrier suitable for parenteral administration. One skilled in the art is well aware of the dosages of RNA or DNA required to induce the production of interferon in animals and to aid protection from a variety of viral infections.

The following Example illustrates the preparation of poly(I:C) microcapsules comprising a poly-(DL-lactide-co-glycolide) (DL-PLG) excipient.

Example

A DL-PLG polymer solution is first prepared by introducing 1.0 g of DL-PLG and 50 g of ethyl acetate (EM Science, Gibbstown, N.J., U.S.A.) into a 4 oz (115 g) glass jar. The DL-PLG has a 53:47 mole ratio of lactide-to-glycolide and an inherent viscosity of 0.91 dL/g as measured at 30°C in hexafluoroisopropanol at a concentration of 0.15 g/dl. A 25 mm Teflon (registered Trade Mark) stir bar is put into the jar and the jar is then sealed with a Teflon-lined scrap cap. The bottle is placed on a hot plate/stirrer. The DL-PLG/ethyl acetate mixture is stirred while heating to 58°C to dissolve the polymer. Once the polymer is dissolved, the jar is removed from the hot plate/stirrer and the DL-PLG solution is allowed to cool to 45°C. Next, 7.5 g of the DL-PLG solution are transferred to a Pyrex test tube (150 mm long, 17 mm outer diameter) containing 100 mg of poly(I:C) (Double strand, physiological salt, lyophilized, Pharmacia/PL Biochemicals, Piscataway, N.J., U.S.A.).

The remainder of the DL-PLG solution is transferred to a 100-ml resin kettle (Knotes Glass Co.,. Vineland, N.J., U.S.A) filled with a true-bore stirshaft, a 38-mm Teflon-turbine impellor (Ace Glass, Inc., Vineland, N.J., U.S.A.), and a Fisher Steadi-speed motor (Fisher Scientific Company, Norcross, Ga, U.S.A.). The solution containing the poly(I:C) is then homogenized at a setting of 5.5 with a Brinkmann Polytron (Brinkmann Instruments, Model PT-10-35 with a PTIOST probe, from Westbury, N.Y., U.S.A.) for four 1-min. bursts with 30 sec. between each burst. The resulting polymer/poly(I:C) suspension is then added to the resin kettle and stirred at 1200 rpm (reverse position). Next, 14 ml of silicone oil (Dow Corning 200 Fluid, 100 C St. Midland, N.J. U.S.A.) are pumped into the resin kettle at a rate of 2 ml/min. After the silicone oil addition is complete, the contents of the resin kettle are quickly poured into 3 litres of heptane (Phillips Chemical Co., Borger, Tx., U.S.A.) contained in a 4-litre beaker and stirred at about 750 rpm with a 50 mm stainless steel impellor. The microcapsules are stirred in the heptane for 45 min. to extract any remaining ethyl acetate The microcapsules are then collected by vacuum filtration, washed with about 250 ml of heptane, and dried in a vacuum chamber maintained at room temperature.

Microcapsules made by the above process contained 0.55 wt % poly(I:C).

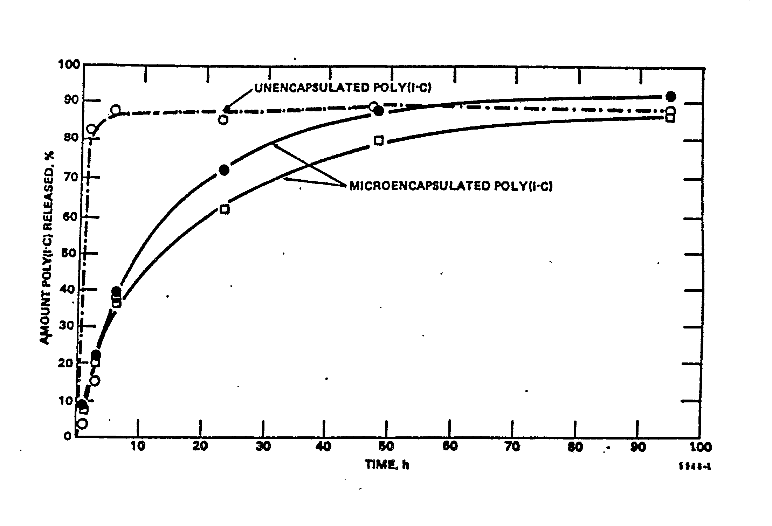

The in vitro release characteristics of these microcapsules were measured. The accompanying drawing shows the in vitro release curves of poly-(I:C) in unencapsulated form and from the microcapsules into a receiving fluid consisting of 0.15 M sodium chloride maintained at 37°C. The amount of poly(I:C) released into the sodium chloride solution was quantified spectrophotometrically at 250 nm. Compared to unencapsulated poly(I:C), the microencapsulated poly(I:C) is released into the aqueous receiving fluid in a controlled manner over a longer period of time. The microcapsules release the poly(I:C) in vitro for up to 118 hours.

**Claims**

1. A composition which comprises RNA and/or DNA encapsulated as microcapsules in a biodegradable, biocompatible polymeric material.

2. A composition according to claim 1, wherein the RNA and/or DNA is RNA.

3. A composition according to claim 1, wherein the RNA and/or DNA is a complex of polyriboinosinic-polyribocytidylic acid.

4 A composition according to any preceding claim, which comprises from 0.01 to 75 wt. % of the RNA and/or DNA based on the polymeric material.

5. A composition according to any of claims 1 to 4, wherein the RNA and/or DNA is double-stranded.

6. A composition according to any of claims 1 to 4, wherein the RNA and/or DNA is single-stranded.

7. A composition according to any preceding claim, wherein the polymer is selected from poly-(D,L-lactide), poly(glycolic acid), copolymers of mixed D,L-lactide and glycolide, copolyoxalates, polycaprolactone, poly(lactide-co-caprolactone), poly(esteramides) and poly(betahydroxy butyric acid).

8. A composition according to claim 3, wherein the polymer is poly(DL-lactide-co-glycolide).

9. A composition according to any preceding claim, which comprises a pharmaceutically acceptable carrier for the microcapsules, the carrier being suitable for parenteral administration to an animal.

10. The use of a composition according to any preceding claim for the manufacture of a medicament for use in protecting an animal from viral infections, the RNA and/or DNA being released at a controlled, predetermined rate to induce the production of interferon and thereby stimulate the immune system of the animal.